# EUROPEAN PATENT SPECIFICATION

(11) **EP 3 250 186 B1**
(45) Date of publication and mention of the grant of the patent: **30.03.2022**
(21) Application number: 16709575.1
(22) Date of filing: 27.01.2016
(51) Int. Cl.: A61K 9/00, A61K 8/365, A61K 8/44, A61K 8/73, A61Q 19/00, A61K 47/36, A61K 31/19, A61K 31/198, A61K 31/205, A61K 9/06, A61Q 19/08, A61K 31/722, A61Q 19/06

(54) **COMPOSITIONS COMPRISING A) CHITOSAN, B) GLYCOLIC ACID, C) CARNITINE AND/OR N-ACETYL CYSTEINE FOR THE DERMAL-EPIDERMAL PEELING TREATMENT**
ZUSAMMENSETZUNGEN MIT A) CHITOSAN, B) GLYKOLSÄURE, C) CARNITIN UND/ODER N-ACETYL-CYSTEIN ZUR DERMALEN-EPIDERMALEN PEELINGBEHANDLUNG
COMPOSITIONS COMPRENANT A) DU CHITOSANE, B) DE L'ACIDE GLYCOLIQUE, C) DE LA CARNITINE ET/OU DE LA N-ACÉTYL-CYSTÉINE UTILISÉES POUR UN TRAITEMENT D'ABRASION DERMIQUE-ÉPIDERMIQUE

(30) Priority: 27.01.2015 IT MI20150084
(43) Date of publication of application: 06.12.2017
(73) Proprietor: Professional Dietetics S.p.A. in forma abbreviata "P.D. S.p.A.", 20129 Milano (IT)
(72) Inventor: GIORGETTI, Paolo Luca Maria, 20129 Milano (IT)
(74) Representative: Bianchetti & Minoja SRL
(86) International application number: PCT/IB2016/050395
(87) International publication number: WO 2016/120796

(56) References cited:
- EP-A2- 0 408 069
- WO-A2-2004/087072
- JP-A- 2005 247 700
- US-A1- 2003 206 958
- US-A1- 2003 229 141
- US-A1- 2006 216 251
- P PERUGINI ET AL: "Study on glycolic acid delivery by liposomes and microspheres", INTERNATIONAL JOURNAL OF PHARMACEUTICS, vol. 196, no. 1, 1 February 2000 (2000-02-01), pages 51-61, XP055125208, ISSN: 0378-5173, DOI: 10.1016/S0378-5173(99)00439-1

## Description

The invention relates to compositions comprising hydroxyacids, acetyl amino acids and chitosan for dermoepidermal peeling treatment.

### Prior art

The conditions known as skin aging and photoaging represent the objective dermatological manifestations of highly complex biochemical phenomena involving the skin cells and the superficial skin tissue structures and, above all, the deep skin tissue structures.

One of the main strategies used to improve signs of aging, such as loss of dermal trophism, loss of volumes, melanic pigmentation and the appearance of fine, deep wrinkles, is to apply chemical peeling preparations. Said preparations, which have been used for decades, consist of aqueous gels or solutions containing high concentrations of acids with exfoliating, dermostimulating and sebostatic activities, supported by substances with a dermoplastic action (retinoids) and a depigmenting action (azelaic acid, kojic acid, phytic acid and ascorbic acid). These preparations, containing alpha-hydroxyacids such as glycolic acid, beta-hydroxyacids such as salicylic acid, beta-keto acids such as pyruvic acid, and halogenated acids such as trichloroacetic acid (TCA), in high percentages by weight, ranging from 25 to 70%, and combinations thereof with phenols such as resorcin, are the main tools used for dermoepidermal chemical peeling.

More recently, dermostimulating peeling treatments, wherein a first preliminary treatment with alpha- and beta-hydroxyacids to induce keratolysis is followed by application of lipogels or pastes containing high concentrations of retinoids and depigmenting substances such as azelaic, kojic, phytic and ascorbic acid, represented a novel and more rational frame of reference for treating skin aging and photoaging syndromes with signs of hyperkeratosis, poor trophism and dermoepidermal plasticity, and melanic pigmentation [1,2]. High concentrations of retinoic acid and retinol palmitate, up to 10% w/w, elicit a significant increase in dermoepidermal tissue regeneration and, at the same time, a great reduction in sebaceous secretion [3,4]. These effects, which are well documented and confirmed by the medical literature and the presence on the market of well-known proprietary anti-acne products, are based on a receptor interaction between the retinoid and specific nuclear receptors which precede hyperexpression of genes. In particular, retinoids have proved to exert a skin-regenerating stimulus action, increasing the synthesis of glycosaminoglycans and dermal connective tissue proteins such as hyaluronic acid, collagen and elastin [5,6].

However, preparations based on acids and retinoids have some major side effects on the skin, due to the extent of their action mechanism: excessive exfoliation, irritation with sometimes marked inflammation and, in the case of longer-term application, symptoms such as frosting (coagulation of the proteins in the superficial and deep dermal regions) and hypertrophic scars [7].

These side effects are often poorly tolerated by patients, due to the skin pain and discomfort which may last for days, and to the necessary avoidance of social and working situations required by said medical practices, in view of the noticeable inflammation and desquamation which makes patients reluctant to be seen in public. In addition to said problems, there are also other difficulties such as increased risk of opportunistic infections such as cold sores or mycosis, caused by a temporary reduction in the local immune defences exerted by said preparations, and adverse events such as reactive hyperpigmentation and burns [7,8], in the event of medical malpractice or drug interactions.

### DESCRIPTION OF THE INVENTION

It has now been found that the drawbacks of the prior art can be overcome by a polyaminoglucosan, in particular chitosan, N-acetyl cysteine and L-carnitine, with exfoliating hydroxyacids.

The object of the invention is therefore topical compositions intended for chemical dermoepidermal peeling, consisting of gelled aqueous solutions comprising chitosan, glycolic acid, N-acetyl-L-cysteine and L-carnitine.

Chitosan therefore takes the form of a polycation, due to the presence of acids that salify glucosamine amino groups.

As well as renewing the epidermis, the compositions according to the invention promote mechanical drainage of melanic pigmentation, reduce its synthesis and induce a sebostatic and comedolytic effect, and are not highly invasive for the patient, as they only cause modest exfoliation and few irritative/inflammatory symptoms.

L-Carnitine, preferably in the form of a chloride, and N-acetyl-L-cysteine contribute to the exfoliating, sebostatic/lipolytic and depigmenting activity. Chitosan, a glucosamine polymer obtained by partial chemical deacetylation of the chitin obtained from marine crustacea, is a film-forming polymer that only becomes water-soluble after protonation of the amino groups of the glucosamine of which it consists. Said protonation, which takes place at a pH less than or equal to 4, makes the chitosan simultaneously water-soluble and able to swell, giving rise to aqueous gels of varying viscosities. Gelling of chitosan in water in the presence of glycolic acid, L-camitine and/or N-acetylcysteine makes the compositions according to the invention particularly practical for topical application (skin adherence), unlike liquid preparations, which tend to drip onto the patient's skin. At the same time, gelling reduces the relative aggressiveness of the individual acids, which penetrate the skin evenly, thus reducing accumulation, which can give rise to rashes. L-carnitine chloride performs a sebostatic and lipolytic action, associated with its known ability to increase the entry of fatty substrates into the cell mitochondrion. Said activity is particularly useful to increase the beta-oxidative use of the fats in adipose tissue in conditions such as seborrhoea and cellulite.

N-Acetylcysteine acts as a melanic depigmenting agent, by switching melanin synthesis by the melanocyte towards pheomelanin (synthesis of the pheomelanin precursors cisteinyl-DOPA and glutathionyl-DOPA) [9], and as a mitochondrion-protecting and antioxidant agent (NAC is the precursor of glutathione). NAC is also a hyaluronidase and dermal elastase inhibitor [10].

Chitosan, after being absorbed through the skin, undergoes gradual degradation in the dermal tissue in the presence of acids, leading to the release of glucosamine monomers and N-acetylglucosamine, substrates which selectively promote the synthesis of dermal hyaluronic acid and act as melanic depigmenting agents [11,12,13].

Chitosan preferably has a mean molecular weight, detennined by gel permeation (Mᵥ), ranging from 10,000 to 250,000 Daltons, and a degree of deacetylation ranging from 70 to 95%. Chitosan is present in concentrations ranging from 0.1 to 2% w/w of the finished preparation.

The concentrations by weight of glycolic acid can range from 30 to 70% w/w, more preferably from 40 to 60% w/w. The L-camitine chloride concentrations can range from 6 to 15% w/w of the finished preparation.

The N-acetylcysteine concentrations can range from 1 to 10% w/w of the finished preparation. Compositions containing both L-carnitine chloride and N-acetyl-L-cysteine are preferred.

Said compositions can also contain preservatives, for example sorbic acid salts such as potassium sorbate, benzoic acid salts such as sodium benzoate, and chelating agents such as phytic acid and EDTA.

The composition of the invention has surprisingly proved to induce an effective epidermolytic, depigmenting, lipolytic and sebostatic action, without giving rise to any significant exfoliation or irritative/inflammatory effects, and significantly improves patient compliance; on average, the isolation time from social and working situations was over 70% less than with peeling preparations containing the same concentration and type of acids. The composition also exhibited excellent manageability, very versatile application to a wide range of aesthetic skin syndromes such as skin aging, melanic hyperpigmentation, seborrhoea, comedonic acne and cellulite, and a high safety level, giving rise to few adverse effects, which are easily managed by the doctor and patient.

The invention is illustrated in greater detail in the examples below.

### EXAMPLE 1

### AGING FACE-NECK PEELING GEL (60% GLYCOLIC ACID)

| Phase | Raw material | g/100 g |
|---|---|---|
| A | Potassium sorbate | 0.20 |
| A | Demineralised water (reverse osmosis) | 0.60 |
| B | N-acetylcysteine | 6.00 |
| B | L-carnitine chloride | 6.00 |
| B | Glycolic acid (70% solution) | 86.2 |
| C | Chitosan (MW=120,000 Daltons, DD^{∗} 85%) | 1.00 |

| | | |
|---|---|---|
| ^{∗}*DD*=*degree of deacetylation* | | |

### Preparation method

Prepare Phase A by dissolving K sorbate under stirring in the available water until completely dissolved. Prepare Phase B by sequentially dissolving N-acetylcysteine and L-carnitine HCl in 70% glycolic acid solution, heating gently to 50°C if necessary and stirring with a magnetic stirrer until completely dissolved (check that NAC has dissolved before adding L-carnitine HCl). Combine Phase A drop by drop with Phase B under stirring. Finally, disperse chitosan (Phase C) under stirring, maintaining t=45°C until complete hydration and a translucent dispersion without undissolved aggregates is obtained. Cool gel and check that the pH is 0.5<pH<0.8.

### EXAMPLE 2

### SEBORRHOEA/ACNE PEELING GEL (40% GLYCOLIC ACID)

| Phase | Raw material | g/100 g |
|---|---|---|
| A | Potassium sorbate | 0.20 |
| A | Demineralised water (reverse osmosis) | 0.60 |
| B | N-acetylcysteine | 2.00 |
| B | L-camitine chloride | 8.00 |
| B | Glycolic acid (70% solution) | 58.0 |
| B | Demineralised water (reverse osmosis) | q.s. for 100 g |
| C | Chitosan (MW=120,000 Daltons, DD^{∗} 85%) | 1.00 |

| | | |
|---|---|---|
| ^{∗}*DD=degree of deacetylation* | | |

### Preparation method

Prepare Phase A by dissolving K sorbate under stirring in the available water until completely dissolved. Prepare Phase B by sequentially dissolving N-acetylcysteine and L-carnitine HCl in the available water; then, when a clear solution without aggregates has been obtained, disperse the 70% glycolic acid solution under stirring until a clear solution is obtained. Combine Phase A drop by drop with Phase B under stirring. Finally, disperse chitosan (Phase C) under stirring, maintaining t=45°C until complete hydration and a translucent dispersion without undissolved aggregates is obtained. Cool gel and check that the pH is 0.5<pH<0.8.

### EXAMPLE 3

### CELLULITE AND ADIPOSITY PEELING GEL (60% GLYCOLIC ACID)

| Phase | Raw material | *g*/ |
|---|---|---|
| A | Potassium sorbate | 0.20 |
| A | Demineralised water (reverse osmosis) | 0.60 |
| B | L-camitine chloride | 10.00 |
| B | Glycolic acid (70% solution) | 86.2 |
| B | N-acetylcysteine | 2.00 |
| C | Chitosan (MW=120,000 Daltons, DD^{∗} 85%) | 1.00 |

| | | |
|---|---|---|
| ^{∗}*DD*=*degree of deacetylation* | | |

### Preparation method

Prepare Phase A by dissolving K sorbate under stirring in the available water until completely dissolved. Prepare Phase B by sequentially dissolving N-acetylcysteine and L-camitine HCl in 70% glycolic acid solution under stirring until a clear solution is obtained. Combine Phase A drop by drop with Phase B under stirring. Finally, disperse chitosan (Phase C) under stirring, maintaining t=45°C until complete hydration and a translucent dispersion without undissolved aggregates is obtained. Cool gel and check that the pH is 0.5<pH<0.8.

### REFERENCES

1. Doris Hexsel, Rosemari Mazzuco, Taciana Dal'Forno & Debora Zechmeister. (2005) Microdermabrasion followed by a 5% retinoid acid chemical peel vs. a 5% retinoid acid chemical peel for the treatment of photoaging - a pilot study. Journal of Cosmetic Dermatology 4:2, 111-116.
2. Mukherjee S, Date A, Patravale V, Korting HC, Roeder A, Weindl G. Retinoids in the treatment of skin aging: an overview of clinical efficacy and safety. Clin Interv Aging. 2006;1(4):327-48.
3. Darlenski R, Surber C, Fluhr JW. Topical retinoids in the management of photodamaged skin: from theory to evidence-based practical approach. Br J Dermatol. 2010 Dec;163(6):1157-65.
4. Mukherjee S, Date A, Patravale V, Korting HC, Roeder A, Weindl G. Retinoids in the treatment of skin aging: an overview of clinical efficacy and safety. Clin Interv Aging. 2006;1(4):327-48.
5. Shigematsu T, Tajima S. Modulation of collagen synthesis and cell proliferation by retinoids in human skin fibroblasts. J Dermatol Sci. 1995 Mar;9(2):142-5.
6. Edward M. Effects of retinoids on glycosaminoglycan synthesis by human skin fibroblasts grown as monolayers and within contracted collagen lattices. Br J Dermatol. 1995 Aug;133(2):223-30.
7. Gerber P, Kukova G, Bölke E, Homey B, Diedrichson E. Severe hyperpigmentation and scarring following glycolic acid peel treatment in combination with low-dose isotretinoin. Eur J Med Res. 2014 Nov 7;19(1):60.
8. Sakai A, Yamamoto Y, Uede K, Furukawa F. Changes of epidermal Langerhans cells in skin treated with trichloroacetic acid. Eur J Dermatol. 2005 Jul-Aug;15(4):239-42.
9. JP Benedetto, JP Ortonne, C Voulot, C Khatchadourian, G Prota and J Thivolet. Role of thiol compounds in mammalian melanin pigmentation: Part I. Reduced and oxidized glutathione. Journal of Investigative Dermatology, Vol 77, 402-405.
10. Sunitha K, Suresh P, Santhosh MS, Hemshekhar M, Thushara RM, Marathe GK, Thirunavukkarasu C, Kemparaju K, Kumar MS, Girish KS. Inhibition of hyaluronidase by N-acetyl cysteine and glutathione: role of thiol group in hyaluronan protection. Int J Biol Macromol. 2013 Apr;55:39-46.
11. Kimball AB, Kaczvinsky JR, Li J, Robinson LR, Matts PJ, Berge CA, Miyamoto K, Bissett DL. Reduction in the appearance of facial hyperpigmentation after use of moisturizers with a combination of topical niacinamide and N-acetyl glucosamine: results of a randomized, double-blind, vehicle-controlled trial. Br J Dermatol. 2010 Feb 1;162(2):435-41.
12. Bissett DL, Robinson LR, Raleigh PS, Miyamoto K, Hakozaki T, Li J, Kelm GR. Reduction in the appearance of facial hyperpigmentation by topical N-acetyl glucosamine. J Cosmet Dermatol. 2007 Mar;6(1):20-6.
13. Po 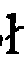 ubinska A, Cwalinski J, Baum E, Br 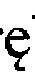 borowicz A. N-Acetylglucosamine modulates function of the skin fibroblasts. Int J Cosmet Sci. 2013 Oct;35(5):472-6.

## Claims

1. Chemical dermoepidermal peeling compositions comprising:
a) glycolic acid;
b) chitosan;
c) N-acetyl cysteine and L-carnitine.

2. Compositions as claimed in claim 1 wherein chitosan has a mean molecular weight, determined by gel permeation, ranging from 10,000 to 250,000 Daltons, and a degree of deacetylation ranging from 70 to 95%.

3. Compositions as claimed in claim 1 or 2 wherein chitosan is present in concentrations ranging from 0.1 to 2% w/w.

4. Compositions as claimed in any one of claims 1 to 3 wherein glycolic acid is present in concentrations ranging from 30 to 70% w/w.

5. Compositions as claimed in any one of claims 1 to 4 wherein L-camitine chloride is present in concentrations ranging from 6 to 15% w/w.

6. Compositions as claimed in any one of claims 1 to 5 wherein N-acetylcysteine is present in concentrations ranging from 1 to 10% w/w.

7. Compositions as claimed in any one of claims 1 to 6 containing N-acetyl-L-cysteine and L-carnitine chloride.

8. Compositions as claimed in any one of claims 1 to 7 further containing preservatives.

9. Compositions as claimed in claim 8 wherein preservatives are selected from potassium sorbate, sodium benzoate and phytic acid and EDTA.

10. Compositions as claimed in any of one claims 1 to 9, in the form of a hydrogel.

11. Use of the compositions claimed in claims 1 to 10 as a medical device or cosmetic product for topical use in humans and animals, for the cosmetic dermoepidermal peeling treatment to treat the main signs of skin aging, acne and seborrhoea, acquired melanic pigmentation, cellulite and local adiposity.

## Patentansprüche

1. Chemische dermo-epidermale Peelingzusammensetzungen, umfassend:
a) Glycolsäure;
b) Chitosan;
c) N-Acetylcystein und L-Carnitin.

2. Zusammensetzungen gemäß Anspruch 1, wobei Chitosan ein durch Gelpermeation bestimmtes mittleres Molekulargewicht im Bereich von 10 000 bis 250 000 Dalton und einen Deacetylierungsgrad im Bereich von 70 bis 95% aufweist.

3. Zusammensetzungen gemäß Anspruch 1 oder 2, wobei Chitosan in Konzentrationen im Bereich von 0,1 bis 2 Gew.-% vorhanden ist.

4. Zusammensetzungen gemäß einem der Ansprüche 1 bis 3, wobei Glycolsäure in Konzentrationen im Bereich von 30 bis 70 Gew.-% vorhanden ist.

5. Zusammensetzungen gemäß einem der Ansprüche 1 bis 4, wobei L-Carnitinchlorid in Konzentrationen im Bereich von 6 bis 15 Gew.-% vorhanden ist.

6. Zusammensetzungen gemäß einem der Ansprüche 1 bis 5, wobei N-Acetylcystein in Konzentrationen im Bereich von 1 bis 10 Gew.-% vorhanden ist.

7. Zusammensetzungen gemäß einem der Ansprüche 1 bis 6, die N-Acetyl-L-cystein und L-Carnitinchlorid enthalten.

8. Zusammensetzungen gemäß einem der Ansprüche 1 bis 7, die weiterhin Konservierungsstoffe enthalten.

9. Zusammensetzungen gemäß Anspruch 8, wobei Konservierurigsstoffe aus Kaliumsorbat, Natriumbenzoat und Phytinsäure und EDTA ausgewählt sind.

10. Zusammensetzungen gemäß einem der Ansprüche 1 bis 9 in Form eines Hydrogels.

11. Verwendung der Zusammensetzungen gemäß den Ansprüchen 1 bis 10 als medizinische Vorrichtung oder kosmetisches Produkt zur topischen Verwendung bei Menschen und Tieren, für die kosmetische dermo-epidermale Peelingbehandlung zur Behandlung der Hauptanzeichen der Hautalterung, von Akne und Seborrhoea, erworbener Melaninpigmentierung, Cellulite und lokaler Fettansammlung.

## Revendications

1. Compositions chimiques de gommage dermo-épidermique, qui comprennent :
a) de l'acide glycolique ;
b) du chitosane ;
c) de la N-acétylcystéine et de la L-carnitine.

2. Compositions selon la revendication 1, dans lesquelles le chitosane possède un poids moléculaire moyen, déterminé par perméation sur gel, qui se situe dans la plage de 10.000 à 250.000 Daltons et un degré de désacétylation qui se situe dans la plage de 70 à 95 %.

3. Compositions selon la revendication 1 ou 2, dans lesquelles le chitosane est présent dans des concentrations qui se situent dans la plage de 0,1 à 2 % en poids/poids.

4. Compositions selon l'une quelconque des revendications 1 à 3, dans lesquelles l'acide glycolique est présent dans des concentrations qui se situent dans la plage de 30 à 70 % en poids/poids.

5. Compositions selon l'une quelconque des revendications 1 à 4, dans lesquelles le chlorure de L-carnitine est présent dans des concentrations qui se situent dans la plage de 6 à 15 % en poids/poids.

6. Compositions selon l'une quelconque des revendications 1 à 5, dans lesquelles la N-acétylcystéine est présent dans des concentrations qui se situent dans la plage de 1 à 10 % en poids/poids.

7. Compositions selon l'une quelconque des revendications 1 à 6, qui contiennent de la N-acétyl-L-cystéine et du chlorure de L-carnitine.

8. Compositions selon l'une quelconque des revendications 1 à 7, qui contiennent en outre des conservateurs.

9. Compositions selon la revendication 8, dans lesquelles les conservateurs sont choisis parmi le sorbate de potassium, le benzoate de sodium et l'acide phytique et le EDTA.

10. Compositions selon l'une quelconque des revendications 1 à 9, sous la forme d'un hydrogel.

11. Utilisation des compositions telles que revendiquées dans les revendications 1 à 10, sous la forme d'un dispositif médical ou d'un produit cosmétique pour une utilisation locale chez l'être humain et l'animal, à des fins de traitement cosmétique par gommage dermo-épidermique, pour le traitement des signes majeurs du vieillissement de la peau, de l'acné et de la séborrhée, de la pigmentation mélanique acquise, de la cellulite et de l'adiposité locale.
